# EUROPÄISCHE PATENTSCHRIFT

(11) **EP 2 833 950 B1**
(45) Veröffentlichungstag und Bekanntmachung des Hinweises auf die Patenterteilung: **04.05.2016**
(21) Anmeldenummer: 13716254.1
(22) Anmeldetag: 05.04.2013
(51) Int. Cl.: A61M 11/00, B05B 7/00, B05B 7/14, B05B 11/06, A61M 15/00, A61M 11/06

(54) **SPENDER ZUR AUSBRINGUNG EINER INSBESONDERE KÖRNIGEN ODER PULVERFÖRMIGEN SUBSTANZ UND VERFAHREN ZUR ENTLEERUNG EINER SUBSTANZ-AUFNAHME**
DISPENSER FOR DISCHARGING AN IN PARTICULAR GRANULAR OR POWDERY SUBSTANCE, AND METHOD FOR EMPTYING A SUBSTANCE RESERVOIR
DISTRIBUTEUR SERVANT À DÉLIVRER UNE SUBSTANCE, EN PARTICULIER GRANULAIRE OU PULVÉRULENTE, ET PROCÉDÉ PERMETTANT DE VIDER UN RÉCIPIENT CONTENANT LADITE SUBSTANCE

(30) Priorität: 05.04.2012 DE 102012103000
(43) Veröffentlichungstag der Anmeldung: 11.02.2015
(73) Patentinhaber: RPC Formatec GmbH, 97638 Mellrichstadt (DE)
(72) Erfinder: KRIEGER, Johannes, 97638 Mellrichstadt (DE)
(74) Vertreter: Müller, Enno
(86) Internationale Anmeldenummer: PCT/EP2013/057166
(87) Internationale Veröffentlichungsnummer: WO 2013/150129

(56) Entgegenhaltungen:
- WO-A1-90/07351
- DE-A1- 19 500 764
- FR-A1- 2 918 299
- US-A- 3 856 185

## Beschreibung

Die Erfindung betrifft zunächst einen Spender zur Ausbringung einer insbesondere körnigen oder pulverförmigen Substanz durch Ausblasen, mit einem Ausblaskanal und einer in den Ausblaskanal mündenden Saugleitung, zum Einsaugen der Substanz in den Ausblaskanal, wobei in einem dem Ausblaskanal vorgeschalteten, in Richtung auf den Ausblaskanal durch ein druckabhängig öffnendes Ventil verschlossenen Kanalabschnitt ein Luft-Überdruck aufbaubar ist, zur druckgesteuerten Ausbildung einer Ausblasströmung in einem Ausblaskanal, wobei weiter Spender eine Aufnahme für ein Substanz-Vorratsteil aufweist und ein Substanz-Vorratsteil, wobei das Substanz-Vorratsteil eine Mehrzahl von Substanz-Aufnahmen aufweist und die Saugleitung eine Mündung aufweist.

Weiter betrifft die Erfindung eine Verwendung eines Spenders nach einem der gegenständlichen Ansprüche und ein Verfahren zur Entleerung eines Spenders nach einem der gegenständlichen Ansprüche.

Aus der WO 90/07351 ist ein Inhalator bekannt, bei dem durch einen Luftkomprimierungskolben ein Blasstrom erzeugt wird, welcher einen, ein medizinisches Pulver ansaugenden, Saugluftstrom erzeugt.

Aus der FR 2 082 780 A ist ein zweistufiges Mahlwerk für Lyophilisationsteile bekannt. In einem Vorratsraum sind zunächst größere Lyophilisationsteile enthalten, die mittels eines Kolbens gegen eine drehende Mahlwerkscheibe gedrückt werden. Die hierdurch erzeugten kleineren Partikel werden nach dem Venturi-Prinzip über eine Leitung in ein zweites Mahlwerk gesaugt. Das Venturi-Prinzip ist durch eine zur entsprechenden Einsaugung unterbrochene

Druckleitung verwirklicht. Darüber hinaus ist zum Stand der Technik die DE 195 00 764 A1 bekannt.

Im Hinblick auf den genannten Stand der Technik beschäftigt sich die Erfindung mit der Aufgabe, einen vorteilhaften Spender zur Ausbringung einer insbesondere körnigen oder pulverförmigen Substanz durch Ausblasen anzugeben.

Wesentlich hierfür ist bei dem Gegenstand des Anspruches 1, dass in dem Kanalabschnitt ein Luft-Komprimierungskolben zwischen einer Ruhe- oder Ausgangsstellung und einer Betätigungsstellung, in welcher er nahe an das Ventil herangefahren ist, bewegbar ist und dass die Saugleitung eine Mündung aufweist, die derart strukturiert ist, dass sie leicht zum Einstechen in eine Substanz-Aufnahme dienen kann, wobei weiter die Substanz-Aufnahme aus einem unteren kugelabschnittsförmigen Behälter besteht, der oberseitig in einer ebenen Fläche durch eine Folie abgedeckt ist.

Durch Komprimieren der Luft in dem Kanalabschnitt kann zunächst, ohne dass bereits eine Saugströmung in dem Saugkanal verursacht ist, ein bestimmter Druckaufbau erreicht werden, der dann bei Öffnen des Ventils sogleich eine günstige hohe Strömung im Ausblaskanal erzeugt, welche die gewünschte Sogwirkung im Saugkanal mit sich bringt und die gewünschte auszubringende Substanz sicher ansaugt und durch den Ausblaskanal ausgeben lässt. Dadurch, dass gleichsam zunächst ein Aufpumpen erfolgen kann, bis die gewünschte Luftströmung sicher erreichbar ist, ist eine solche Ausgestaltung insbesondere als Handgerät geeignet. Es kann mit Fingerdruck bspw. ein Luft-Komprimierungskolben so weit bewegt werden, dass sich der gewünschte Überdruck ergibt und das Ventil öffnet und sich dann der beschriebene Effekt günstig einstellt.

Hinsichtlich des Verfahrens schlägt die Erfindung vor, dass mit Hilfe eines Spenders wie vorbeschrieben oder in weiterer hier beschriebenen Ausgestaltung die Substanz aus der Substanz-Aufnahme, wobei die Substanz in Form von Körnchen eines Durchmessers von 0,1 mm bis 2 mm vorliegt, und mittels eines in dem durch Fingerdruck durch Bewegen des Luft-Komprimierungskolbens erzeugten Blasstrom mündenden Saug-Luftstroms angesaugt und ausgegeben wird, wobei der Blasstrom durch Bewegung des Luft-Komprimierungskolbens, soweit, dass sich ein gewünschter Überdruck ergibt und das Ventil öffnet, erzeugt wird.

Hinsichtlich der Verwendung schlägt die Erfindung vor, dass der genannte Spender mit dem durch Fingerdruck bewegbaren Luft-Komprimierungskolben zur Erzeugung eines gewünschten Überdrucks, bei welchem das Ventil öffnet und bei welchem beim Öffnen des Ventils sogleich eine günstig hohe Strömung in dem Ausblaskanal erzeugt wird, welche eine gewünschte Sogwirkung in der Saugleitung mit sich bringt, zur Entleerung mittels der Saugleitung der eine gefriergetrocknete Substanz aufweisenden Substanz-Aufnahme verwendet wird, wobei die Substanz in Form von Körnchen eines Durchmessers von 0,1 mm bis 2 mm vorliegt.

Insbesondere handelt es sich um im medizinischen oder biologischen Bereich gegebene Substanzen, die in kleinen Substanz-Aufnahmen, etwa blisterverschlossenen Kavitäten aufgenommen sind. Die Substanzaufnahme kann auch vergleichsweise klein ausgebildet sein, also etwa bezogen auf eine Kugel, wobei die tatsächliche Geometrie nicht kugelförmig zu sein braucht, ein Volumen aufweisen, das im Hinblick auf die genannte Kugel einem Kugeldurchmesser von 1 mm bis 10 mm bspw. entspricht.

Gerade in Bezug auf solche vergleichsweise geringe Mengen und geringe Größen der Substanz-Aufnahme ist erkannt worden, dass ein solcher Spender, bei dem zudem die erforderliche Saug- und Blasströmung noch durch einen Handbetrieb dann erzeugt werden kann, besonders günstig eingesetzt werden kann. Im Hinblick auf das Ventil ist es bspw. bevorzugt, dass es sich um ein Kunststoffventil handelt. Es kann sich insbesondere um ein selbstschließendes Ventil handeln, wie es etwa aus der DE 19613130 A1 oder der DE 19612 561 A1 bekannt ist.

Bezogen auf die Darstellung der Fig. 4 in der DE 196 12 561 A1, welches das Nachsaugen darstellt, ist ersichtlich, dass das Ventil nach unten gleichsam "durchgebrochen" ist. Durch die obere Öffnung mit dortigem Bezugszeichen 4 wird Luft angesaugt, die in das Behältnis dann durch das geöffnete Ventil einströmt. Dieses Ventil, das im Ruhezustand eine Geometrie etwa gemäß Fig. 2 einnimmt, öffnet nicht im Sinne der Fig. 4 sogleich bei einem ersten Saug-Unterdruck in dem Behältnis, sondern erst, wenn ein gewisses Unterdruckniveau aufgebaut ist, wonach es dann "durchbricht". Diese Eigenschaft ist im hier gegebenen Zusammenhang günstig genutzt, da durch die entsprechende Gestaltung der Geometrie, etwa die Dicken der Ventilwände, günstig eingestellt werden kann, bei welchem Überdruck das Ventil durchbricht.

Insbesondere ist bevorzugt, dass das Ventil einen unteren Halterungsrand und eine obere, sich im Wesentlichen konkav erstreckende Verschlussdecke aufweist, wobei weiter die Verschlussdecke und der Halterungsrand durch eine Verbindungswand verbunden sind. Die Verbindungswand kann konisch oder auch zylindrisch verlaufen. Bezogen auf den Spritzzustand kann der Einbauzustand ungestülpt bzgl. der Verschlussmembran und der Verbindungswandsein. Dies kann zu einer besonderen Spannung in der Verschlussdecke führen. Hierbei kann die Verschlussdecke randseitig auch eine größere Dicke aufweisen als die Verbindungswand. Die Verschlussdecke kann sich ausgehend von ihrem Randbereich kontinuierlich nach innen verjüngen. Die Verbindungswand ist bzgl. einer im Querschnitt erscheinenden Randkante der Verschlussdecke im oberen Bereich angebunden - wiederum bezogen auf den Einbauzustand - und ein unterer freier Randbereich der Randkante der Verschlussdecke ist von der Begrenzungswand im Einbauzustand jedenfalls bevorzugt überfangen.

In weiterer Einzelheit kann ein solches Ventil bzgl. üblicher Spender, bekannt sich bspw. Honigspender, derart eingebaut werden, dass der Durchbruch des Ventils und damit die Erzeugung der Luftströmung im Ausblaskanal dem üblichen Spendevorgang entspricht. Es kann aber auch vorgesehen sein, dass das Ventil umgekehrt eingebaut ist, dass also der Durchbruch gleichsam bezogen auf einen bspw. Honigspender dem Nachsaugen von Luft entspricht.

Alternativ hierzu kann es sich bei dem Ventil auch um ein übliches federbelastetes Kugelventil handeln. Die Feder erbringt eine Vorspannung, die in gleicher Weise ein Öffnen in Abhängigkeit eines bestimmten Überdrucks erst ermöglicht.

Der Spender weist eine Aufnahme für ein Substanz-Vorratsteil auf, welches Substanz-Vorratsteil seinerseits eine Mehrzahl von Substanz-Aufnahmen aufweist. Es kann sich um ein Blisterteil handeln, etwa um ein kreisförmiges, insbesondere kreisringförmiges Blisterteil, das in Kreisform eine Mehrzahl von Blisteraufnahmen hintereinander aufweist. Insbesondere ist das Substanz-Vorratsteil auch kreisscheibenförmig gebildet.

Hinsichtlich des Verfahrens wird mit der Handbetätigung, mittels des von Hand betätigten Pumpkolbens, der Luftdruck aufgebaut, der zur Entleerung einer Substanzaufnahme erforderlich ist, nämlich in dem Sinne, dass der aufgebaute Luftdruck in dem einen vergleichsweise kleinen Durchmesser aufweisenden Ausblaskanal die erforderliche Luftströmung erbringt, um durch den in den Ausblaskanal mündenden Saugkanal das Herausfördern der Substanz aus der Substanzaufnahme und das Einfördern in den Ausblaskanal sicher zu gewährleisten.

Die vor- und nachstehend angegebenen Bereiche bzw. Wertebereiche schließen hinsichtlich der Offenbarung auch sämtliche Zwischenwerte ein, insbesondere in 1/10-Schritten der jeweiligen Dimension, ggf. also auch dimensionslos, bspw. für ein Durchmesserverhältnis, das mit 0,8 bis 0,3 angegeben ist, also auch 0,79 bis 0,3 oder 0,8 bis 0,31 oder 0,79 bis 0,31 etc., einerseits zur Eingrenzung der genannten Bereichsgrenzen von unten und/ oder oben, alternativ oder ergänzend aber auch im Hinblick auf die Offenbarung eines oder mehrerer singulärer Werte aus dem jeweilig angegebenen Bereich.

Nachstehend ist die Erfindung anhand der beigefügten Zeichnung, die jedoch lediglich ein Ausführungsbeispiel darstellt, erläutert. Hierbei zeigt die
einzige Figur: einen Querschnitt durch den Spender und zugeordnete Substanzaufnahme.

Dargestellt und beschrieben ist ein Spender 1 zur Ausbringung einer bspw. körnigen Substanz 2, die in einer Substanz-Aufnahme 3 angeordnet ist. Der Spender 1 weist einen Ausblaskanal 4 und eine Saugleitung 5 auf, die in den Ausblaskanal 4 mündet. Vorgeschaltet in Ausblasrichtung zu dem Ausblaskanal 4 ist ein Ventil 6 in einem Kanalabschnitt 7 angeordnet, das zunächst, im dargestellten Ausgangszustand, verschlossen ist. Weiter weist der Spender 1 einen Luft-Komprimierungskolben 8 auf, der in dem Kanalabschnitt 7 zwischen einer Ruhe- oder Ausgangsstellung, wie dargestellt, und einer Betätigungsstellung, in der er nahe an das Ventil 6 herangefahren ist, bewegbar ist. Mit dem Heranfahren des Luft-Komprimierungskolbens 8 an das Ventil 6 baut sich zwischen dem Luft-Komprimierungskolben 8 und dem Ventil 6 ein Luft-Überdruck auf, bis das Ventil 6 öffnet. Mit Öffnen des Ventils 6 fließt ein vergleichsweise starker Luftstrom durch den Ausblaskanal 4, so dass es bei der Einmündung der Saugleitung in den Ausblaskanal 4 zu einem Saugeffekt kommt und Luft durch die Saugleitung 5 in den Ausblaskanal 4 gesaugt wird. Da die Saugleitung 5 in einem solchen Spendezustand in die Substanz-Aufnahme 3 hineinragt, wird so Substanz 2 aus der Substanz-Aufnahme 3 ausgesaugt und nach Passieren des Mündungsbereiches der Saugleitung 5 in den Ausblaskanal in dem Ausblaskanal 4 in Richtung des Pfeiles P ausgetragen.

Der Luft-Komprimierungskolben 8 kann endseitig eine Betätigungsfläche 9 aufweisen, zur Handbetätigung des Spenders 1. Insbesondere kann der Spender 1 in seiner dargestellten Ausgangsstellung eine Höhe bzw. Erstreckung in Verschieberichtung des Luft-Komprimierungskolbens 8 aufweisen, die bspw. einer zweifachen Handbreite entspricht oder geringer ist, also jedenfalls an die Betätigung von Hand größenmäßig angepasst ist.

Weiter weist der Luft-Komprimierungskolben 8 vorderseitig eine oder zwei Dichtlippen 10 auf, die mit einer Innenfläche 11 des Kanalabschnitts 7 in Berührung sind und so den Aufbau des Luft-Überdrucks beim Verfahren, bezogen auf die Darstellung: Herunterfahren, des Luft-Komprimierungskolbens 8 ermöglichen.

Das Ventil 6 ist bevorzugt ein Kunststoffventil. Es weist bevorzugt einen Einspannflansch 12 auf, mit dem es dichtend im Gehäuse des Spenders 1 gefasst ist. Beim Ausführungsbeispiel weist der Spender 1 hierzu ein Oberteil 13 und ein Unterteil 14 auf, die beide einen Teil des Kanalabschnitts 7 bilden. Ausgehend von dem Einspannflansch 12 weist das Ventil weiter eine Verbindungswand in Form bevorzugt eines Zylinderabschnittes 15 auf, wobei das Ventil beim Ausführungsbeispiel so angeordnet ist, dass sich der Zylinderabschnitt ausgehend von dem Einspannflansch 12 in Richtung auf den Luft-Komprimierungskolben 8 erstreckt. Der Zylinderabschnitt 15 geht sodann in eine Verschlussdecke 16 über, die beim Ausführungsbeispiel und bevorzugt gewölbeartig gebogen verläuft. Hierbei ist die Verschlussdecke 16 dem Luft-Komprimierungskolben 8 zugewandt konvex gewölbt. Die Verschlussdecke 16 weist weiter einen oder mehrere Schlitze auf, wobei die Schlitzwände aber im Ruhezustand, wie in der Figur dargestellt, aneinander liegen.

Im Zuge der Druckerhöhung, wenn der Luft-Komprimierungskolben 8 in Richtung auf das Ventil 6 verfährt, wird aufgrund der genannten Gewölbestruktur zunächst ein näheres Anpressen der Schlitzwände aneinander erreicht, so dass sich sogar die Dichtwirkung des Ventils zunächst noch tendenziell verstärkt.

Dies geschieht solange, bis die Gewölbestruktur dem erhöhten Druck nicht mehr standhält und nach unten, in Richtung des Pfeils P, umstülpt, wobei auch ein Teil des Zylinderabschnitts 15 mit umstülpen kann. Hierbei kommt es dann zu einem Öffnen, Aufklaffen, der Schlitzwände und die Luft strömt unter erhöhtem Druck und damit auch entsprechender Strömungsgeschwindigkeit in den Ausblaskanal 4.

In weiterer Einzelheit weist der Kanalabschnitt 7 einen ersten Durchmesser D₁ auf, während der Ausblaskanal einen zweiten Durchmesser D₂ aufweist. Der zweite Durchmesser D₂ ist kleiner als der erste Durchmesser D₁. Bevorzugt ist ein Verhältnis von 0,8 bis 0,3 bezogen auf D₂ : D₁.

Weiter ist von Bedeutung, dass der Ausblaskanal 4 im Bereich der Einmündung der Saugleitung 5 gegenüber dem Durchmesser D₂ nochmals eine Verjüngung aufweist, mit einem Durchmesser D₃. Der Durchmesser D₃ ist nochmals bevorzugt im gleichen Verhältnisbereich wie D₂ zu D₁ verjüngt, also entspricht auch D₃ : D₂ etwa 0,8 bis 0,3.

Soweit von Durchmesser gesprochen ist, ist davon ausgegangen, dass es sich jeweils um einen freien Kreisquerschnitt in den genannten Abschnitten handelt. Es könnten aber auch hiervon abweichende Querschnitte gegeben sein. Insoweit bezieht sich dann "Durchmesser" auf ein jeweiliges größtes Innenmaß.

Die Saugleitung 5 weist einen Durchmesser D₄ auf. D₄ kann zunächst D₃ entsprechen. Er kann aber auch größer als D₃ oder kleiner als D₃ sein. Bevorzugt ist, dass D₄ kleiner als D₂ aber größer D₃ ist.

Die Saugleitung 5 ist ersichtlich im Querschnitt winkelförmig gebildet, mit einem ersten Horizontalabschnitt 17 und einem zweiten Vertikalabschnitt 18.

Der Winkel zwischen den Abschnitten 17 und 18 braucht kein rechter Winkel zu sein. Beim Ausführungsbeispiel ist allerdings ein rechter Winkel gegeben.

Die Saugleitung 5 bzw. beim Ausführungsbeispiel konkret der Vertikalabschnitt 18 weist endseitig eine Mündung 19 auf, die bevorzugt auch derart strukturiert ist, dass sie leicht zum Einstechen in eine Substanz-Aufnahme 3 dienen kann. Sie kann bspw. einen Öffnungen aufweisenden Spitzenkegel aufweisen. Die Substanz-Aufnahme 3 besteht ersichtlich bevorzugt aus einem unteren kugelabschnittsförmigen Behälter, der oberseitig, in einer ebenen Fläche 20 durch eine Folie abgedeckt ist. Es handelt sich um eine übliche Blister-Verpackung.

Mit der genannten Mündung 19 kann nun die Saugleitung 5 in eine Blisterkavität durch Durchstoßen der Folie einstechen und die Substanz 2 liegt damit dann im Saugstrom, wenn der Spender 1 betätigt wird.

Die Substanz-Aufnahmen 3 sind weiter ersichtlich bevorzugt in einem kreisringförmigen Trägerteil 21 angeordnet, das oberseitig die genannte Folienabdeckung aufweist und unterseitig die Substanz-Aufnahmen 3. Ersichtlich können in einem solchen Trägerteil bspw. fünf bis fünfzig oder mehr Substanzaufnahmen 3 nebeneinander angeordnet und über den Kreisring gleichförmig verteilt ausgebildet sein.

Nach dem Aussaugen einer Substanz-Aufnahme 3 wird das Trägerteil in Kreisumfangsrichtung weiter bewegt, so dass die benachbarte nächste Substanz-Aufnahme 3, oder, wenn aus bestimmten Gründen bevorzugt, eine weiter entfernte Substanz-Aufnahme 3, dann beim nächsten Aussaugen zur Verfügung steht. Hierbei kann zunächst eine Absenkung des Trägerteils erfolgen, dann eine Drehung in Kreisrichtung und sodann, wenn die Ausrichtung einer weiteren Substanz-Aufnahme 3 zu einer Spitze der Saugleitung gegeben ist, wieder ein Anheben, wobei zugleich das Eindringen der Spitze der Saugleitung in die Substanz-Aufnahme 3 erfolgt.

Der Träger 21 kann in einem Gehäuseteil aufgenommen sein, derart, dass er im Wesentlichen bei Benutzung der Vorrichtung nicht sichtbar ist. Hierin kann jedoch ein Fenster ausgebildet sein, bei dem man bspw. die nach einem entsprechenden Vorrücken jeweils zuvor ausgesaugte Substanz-Aufnahme 3 zu Kontrollzwecken sehen kann.

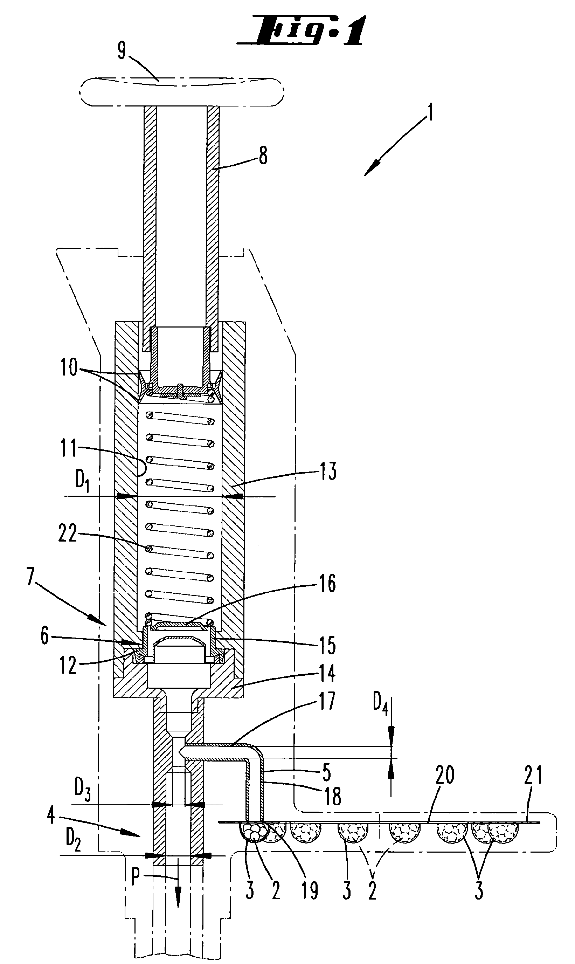

Der Luft-Komprimierungskolben 8 kann in seiner Ausgangsstellung gemäß Fig. 1 ersichtlich noch durch eine Druckfeder 22 vorgespannt sein.

**Bezugszeichenliste**

| | | | |
|---|---|---|---|
| 1 | Spender | P | Pfeil |
| 2 | Substanz | D₁ | erster Durchmesser |
| 3 | Substanz-Aufnahme | D₂ | zweiter Durchmesser |
| 4 | Ausblaskanal | D₃ | dritter Durchmesser |
| 5 | Saugleitung | D₄ | vierter Durchmesser |
| 6 | Ventil | | |
| 7 | Kanalabschnitt | | |
| 8 | Luft-Komprimierungskolben | | |
| 9 | Betätigungsfläche | | |
| 10 | Dichtlippe | | |
| 11 | Innenfläche | | |
| 12 | Einspannflansch | | |
| 13 | Oberteil | | |
| 14 | Unterteil | | |
| 15 | Zylinderabschnitt | | |
| 16 | Membranabschnitt | | |
| 17 | Horizontalabschnitt | | |
| 18 | Vertikalabschnitt | | |
| 19 | Mündung | | |
| 20 | Fläche | | |
| 21 | Träger | | |
| 22 | Druckfeder | | |

## Patentansprüche

1. Spender (1) zur Ausbringung einer insbesondere körnigen oder pulverförmigen Substanz (2) durch Ausblasen, mit einem Ausblaskanal (4) und einer in den Ausblaskanal (4) mündenden Saugleitung (5), zum Einsaugen der Substanz (2) in den Ausblaskanal (4), wobei in einem dem Ausblaskanal (4) vorgeschalteten, in Richtung auf den Ausblaskanal (4) durch ein druckabhängig öffnendes Ventil (6) verschlossenen Kanalabschnitt (7) ein Luft-Überdruck aufbaubar ist, zur druckgesteuerten Ausbildung einer Ausblasströmung in dem Ausblaskanal (4), wobei weiter in dem Kanalabschnitt (7) ein Luft-Komprimierungskolben (8) zwischen einer Ruhe- oder Ausgangsstellung und einer Betätigungsstellung, in der er nah an das Ventil (6) herangefahren ist, bewegbar ist, wobei der Spender (1) eine Aufnahme für ein Substanz-Vorratsteil aufweist und ein Substanz-Vorratsteil, wobei das Substanz-Vorratsteil eine Mehrzahl von Substanz-Aufnahmen (3) aufweist und dass die Saugleitung (5) eine Mündung (19) aufweist, die derart strukturiert ist, dass sie leicht zum Einstechen in eine Substanz-Aufnahme (3) dienen kann, wobei weiter die Substanz-Aufnahme (3) aus einem unteren kugelabschnittsförmigen Behälter besteht, der oberseitig in einer ebenen Fläche (20) durch eine Folie abgedeckt ist.

2. Spender nach Anspruch 1, **dadurch gekennzeichnet, dass** die Substanzaufnahme (3) in einem kreisförmigen Trägerteil (21) ausgebildet ist und dass das Trägerteil (21) in Kreisumfangsrichtung bewegbar ist, so dass eine benachbarte nächste Substanz-Aufnahme (3), oder eine weiter entfernte Substanz-Aufnahme (3), bei einem nächsten Ansaugen zur Verfügung steht.

3. Spender nach einem der Ansprüche 1 oder 2, **dadurch gekennzeichnet, dass** die Saugleitung (5) im Längsschnitt winkelförmig gebildet ist, mit einem ersten Horizontalabschnitt (17) und einem zweiten Vertikalabschnitt (18).

4. Spender nach einem der Ansprüche 1 bis 3, **dadurch gekennzeichnet, dass** das Ventil (6) ein Kunststoffventil ist.

5. Verwendung eines Spenders nach einem der vorstehenden Ansprüche 1 bis 4 mit einem durch Fingerdruck bewegbaren Luft-Komprimierungskolben (8) zur Erzeugung eines gewünschten Überdrucks, bei welchem das Ventil (6) öffnet und bei welchem beim Öffnen des Ventils (6) sogleich eine günstig hohe Strömung in dem Ausblaskanal (4) erzeugt wird, welche eine gewünschte Sogwirkung in der Saugleitung (5) mit sich bringt, zur Entleerung mittels der Saugleitung (5) der eine gefriergetrocknete Substanz (2) aufweisenden Substanz-Aufnahme (3), wobei die Substanz (2) in Form von Körnchen eines Durchmessers von 0,1 bis 2 mm vorliegt.

6. Verfahren zur Entleerung einer eine gefriergetrocknete Substanz aufweisenden Substanz-Aufnahme mit Hilfe eines Spenders nach einem der Ansprüche 1 bis 4, **dadurch gekennzeichnet, dass** die Substanz aus der Substanz-Aufnahme, wobei die Substanz in Form von Körnchen eines Durchmessers von 0,1 bis 2 mm vorliegt, mittels eines in dem durch Fingerdruck durch Bewegen des Luft-Komprimierungskolbens (8) erzeugten Blasstrom mündenden Saugluftstroms angesaugt und ausgegeben wird, wobei der Luft-Komprimierungskolber (8), so weit bewegt werden kann dass sich ein gewünschter Überdruck ergibt und das Ventil (6) öffnet.

7. Verfahren nach Anspruch 6, **dadurch gekennzeichnet, dass** zunächst eine Absenkung des Trägerteils erfolgt, dann eine Drehung in Kreisrichtung und sodann, wenn die Ausrichtung einer weiteren Substanz-Aufnahme (3) zu einer Spitze der Saugleitung (5) gegeben ist, wieder ein Anheben, wobei sogleich das Eindringen der Spitze (19) der Saugleitung (5) in die Substanz-Aufnahme (3) erfolgt.

## Claims

1. A dispenser (1) for discharging an in particular granular or powdery substance (2) by blow-out, with a blow-out duct (4), and with a suction line (5) opening into the blow-out duct (4) for sucking the substance (2) into the blow-out duct (4), wherein a positive air pressure can be built up in a duct section (7) arranged upstream of the blow-out duct (4) and closed in the direction of the blow-out duct (4) by a valve (6) opening in a pressure-dependent manner, for pressure-controlled formation of a blow-out stream in the blow-out duct (4), wherein moreover an air compression piston (8) is movable in the duct section (7) between a rest or start position and an actuation position, in which it is moved close to the valve (6), wherein the dispenser (1) has a seat for a substance storage part, and a substance storage part, wherein the substance storage part has a plurality of substance reservoirs (3), and that the suction line (5) has a mouth (19), which is structured in such a way that it can easily serve to pierce a substance reservoir (3), wherein moreover the substance reservoir (3) consists of a lower container in the shape of a portion of a sphere, which container is closed at the top by a film in a plane surface (20).

2. The dispenser as claimed in claim 1, **characterized in that** substance reservoir (3) is formed in a circular support part (21), and **in that** the support part (21) is movable in the circumferential direction of the circle, such that a next adjacent substance reservoir (3), or a farther away substance reservoir (3), is available for a subsequent suctioning.

3. The dispenser as claimed in either of claims 1 and 2, **characterized in that** the suction line (5) is formed at an angle in the longitudinal section, with a first horizontal portion (17) and a second vertical portion (18).

4. The dispenser as claimed in one of claims 1 through 3, **characterized in that** the valve (6) is a plastic valve.

5. Use of a dispenser as claimed in one of the preceding claims 1 to 4, with an air compression piston (8) movable by finger pressure for generating a desired positive pressure, in which the valve (6) opens and in which, when the valve (6) opens, a high flow rate is immediately generated in the blow-out duct (4), which brings with it a desired sucking action in the suction line (5), for emptying, via the suction line (5), the substance reservoir (3) having a freeze-dried substance (2), wherein the substance (2) is present in the form of grains with a diameter of 0.1 mm to 2 mm.

6. A method for emptying a substance reservoir having a freeze-dried substance, with the aid of a dispenser as claimed in one of claims 1 through 4, **characterized in that** the substance from the substance reservoir, the substance being present in the form of grains with a diameter of 0.1 to 2 mm, is sucked out by means of a suction air stream opening into the blowing stream generated by pressing with a finger and moving the air compression piston (8), and is dispensed, wherein the air compression piston (8) can be moved until a desired positive pressure arises and the valve (6) opens.

7. The method as claimed in claim 6, **characterized in that** the support part is first lowered, then rotated in a circular direction and, when a further substance reservoir (3) is oriented with respect to a tip of the suction line (5), is raised again, with the tip (19) of the suction line (5) immediately penetrating the substance reservoir (3).

## Revendications

1. Distributeur (1) pour délivrer une substance, en particulier granulaire ou pulvérulente, par soufflage, avec un canal d'éjection par soufflage (4) et un conduit d'aspiration (5) débouchant dans le canal d'éjection (4) lequel conduit d'aspiration sert à aspirer la substance (2) dans le canal d'éjection (4), dans lequel une surpression d'air peut être générée dans un tronçon de canal (7) agencée en amont du canal d'éjection (4) lequel est obturé en direction du canal d'éjection (4) par une valve (6) à ouverture dépendant de la pression, pour former un courant de soufflage dans le canal d'éjection (4) par commande en pression, dans lequel en outre un piston de compression d'air (8) est déplaçable dans la tronçon de canal (7) entre une position de repos ou de départ et une position d'actionnement dans laquelle il est avancé à proximité de la valve (6), dans lequel le distributeur (1) présente une partie de réception pour une pièce formant réservoir de substance et une pièce formant réservoir de substance, dans lequel la pièce formant réservoir de substance présente une pluralité de réceptacles de substance (3), le conduit d'aspiration (5) présentant une embouchure (19) qui est structurée pour pouvoir être facilement piqué dans un réceptacle de substance, le réceptacle de substance étant en outre formé par un récipient ayant une portion inférieure sphérique et qui est recouvert du côté supérieur par un film suivant une surface plane (20).

2. Distributeur selon la revendication 1, **caractérisé en ce que** le réceptacle de substance (3) est formé dans une partie de support (21) de forme circulaire et que la partie de support (21) est déplaçable en direction circonférentielle de manière à ce qu'un réceptacle de substance voisin suivant (3) ou un réceptacle de substance plus éloigné (3) soit mis à disposition lors d'une prochaine aspiration.

3. Distributeur selon la revendication 1 ou 2, **caractérisé en ce que** le conduit d'aspiration (5) forme un coude en coupe longitudinale, avec un premier tronçon horizontal (17) et un deuxième tronçon vertical (18).

4. Distributeur selon l'une des revendications 1 à 3, **caractérisé en ce que** la valve (6) est une valve en matière plastique.

5. Utilisation d'un distributeur selon l'une des revendications 1 à 4 ayant un piston de compression d'air (8) actionnable par pression de doigt pour générer une surpression souhaitée, avec laquelle la valve (6) s'ouvre et avec laquelle un courant d'air élevé approprié est généré dans le canal d'éjection (4) immédiatement lors de l'ouverture de la valve (6) lequel entraîne une dépression souhaitée dans le conduit d'aspiration (5) pour vider au moyen du conduit d'aspiration (5) le réceptacle de substance contenant une substance lyophilisée (2) laquelle est sous la forme de grains d'un diamètre allant de 0,1 à 2 mm.

6. Procédé de vidage d'un réceptacle de substance contenant une substance lyophilisée au moyen d'un distributeur selon l'une des revendications 1 à 4, **caractérisé en ce que** la substance laquelle est sous forme de grains d'un diamètre allant de 0,1 à 2 mm, est aspirée et délivrée à partir du réceptacle de substance par un courant d'air d'aspiration débouchant dans le courant de soufflage généré par le déplacement du piston de compression d'air (8) provoqué par pression de doigt, dans lequel le piston de compression d'air (8) peut être déplacé suffisamment loin pour générer une surpression souhaitée et ouvre la valve (6).

7. Procédé selon la revendication 6, **caractérisé en ce que** tout d'abord a lieu un abaissement de la partie de support, puis une rotation dans la direction circulaire et ensuite à nouveau un relèvement lorsqu'un autre réceptacle de substance (3) est aligné avec une pointe du conduit d'aspiration (5), la pointe (19) du conduit d'aspiration (5) pénétrant aussitôt dans le réceptacle de substance (3).
